# EUROPEAN PATENT APPLICATION

(11) **EP 1 403 274 A1**
(43) Date of publication of application: **31.03.2004**
(21) Application number: 02292404.7
(22) Date of filing: 30.09.2002
(51) Int. Cl.: C07K 1/14, C12P 21/02

(54) **Process for the purification of recombinant proteins from complex media and purified proteins obtained thereby**

(71) Applicant: MERISTEM THERAPEUTICS, 63100 Clermont-Ferrand (FR)
(72) Inventor: Bleuart, Sylvie, 63100 Clermont-Ferrand (FR); Boulis, Yannick, 63960 Veyre Monton (FR); Testud, Olivier, 63000 Clermont-Ferrand (FR); Larché-Scrivant, Lydie, 63000 Clermont-Ferrand (FR); Mison, Dominique, 63000 Clermont-Ferrand (FR); Devillers, Claire, 59240 Dunkerque (FR)
(74) Representative: Richebourg, Michel François

(57) **Abstract**

The present invention concerns a method for prepurifying and concentrating a desired recombinant protein from a complex media, wherein said method comprises the steps of bringing the complex media into contact with at least one diatomaceous earth for a sufficient time to effect adsorption of the desired recombinant protein onto the diatomaceous earth and desorbing the desired prepurified and concentrated recombinant protein from the diatomaceous earth.

## Description

The present invention concerns the field of protein purification in general, and in particular recombinant proteins, present in a complex media, that is to say, a media rich in solid particles and/or in various impurities, such as undesirable proteins, nucleic acids, lipids, polyphenols, pigments, lipopolysaccharides, secondary metabolites, polysaccharides, virii and the like. Suitable examples of complex media are plant extracts, mammalian tissue extracts, prokaryotic or eukaryotic cell lysates or hydrolysates, and culture media obtained in bioreactors.

In a process that enables purification of a target molecule or a molecule of interest, the present invention can be advantageously used to obtain an extract that is totally free of solid particles, whilst at the same time, enabling a concentration of the sample and a certain degree of prepurification, notably through the elimination of a significant quantity of impurities. Obtaining such a prepurified extract is quite important with regard to the further optional complementary processing steps, such as liquid chromatography, thereby enabling improved purification of the target molecule or molecule of interest through reduced clogging and consequently increased capacity of the chromatography or separation column. Since the technique of the invention is easy to put into practice and is very cost effective, it is well adapted to industrial scale production without any major disadvantages, especially in terms of system costs, polluting liquid effluents, and the like.

### State of the Art

Generally, a process for purifying a target molecule, and in particular a protein, can be split into 4 main steps :
- a clarification step
- a capture step
- a purification step
- a polishing step

The capture, purification and polishing steps are traditionally carried out using liquid phase chromatography. As regards the first step, known as the clarification step, several different possibilities are available :
- deep filtration
   - centrifugation or decanting
   - ultrafiltration or cross flow microfiltration

Such techniques enable separation of solid particles present in aqueous media. On an industrial scale however, these techniques require costly systems to be put into place.
Moreover, where complex media are involved, in particular where small solid particles or polydisperse particulates are present, decanting does not enable easy preparation of a perfectly clear extract, that is to say, exempt of solid particles. Consequently, several passes on this type of system are often necessary, with different conditions at each pass, in order to obtain the required result.

### Filtration using diatomaceous earth

This material is used in the food industry, such as in brewing and wine production, and in the chemical, pharmaceutical and metallurgical industries as a filtration adjuvant. In these instances, only the filtration function or property of the earth is applied and any adsorption involved solely used to eliminate impurities, without regard to any desorption steps. The earth is most often used as a pre-layer, or added to a liquid extract (slurrying) followed by a supplementary filtration step, and more generally as a combined pre-layer/slurrying step.

### Clarification and concentration/Filtration (filtration cascade)

Filtration enables clarification and concentration of the extract containing the target molecule or molecule of interest. For complex extracts, it is necessary to establish a cascading filtration system, starting from a rough filtration to eliminate the particles of highest granulometry. followed by subsequent steps of finer and finer filtration, and then, once a clear liquid extract has been obtained, one or more ultrafiltration steps to concentrate the molecule of interest. However, on a industrial scale, such a system rapidly becomes too costly, and presents a certain number of technical difficulties, such as the requirement to precisely define the various differing filtration conditions, such as flow rate and turbidity. In addition, there is a greatly increased risk of losing the molecule of interest through non-specific adsorption on the membranes that are used. Another problem with such a cascading system is the length of time that it takes to carry out, especially when one includes membrane regeneration, in situ cleaning and in situ sterilization steps, into the process.

### Precipitation with precipitating agents

This technique is still the most frequently used as a first purification step. Many precipitating agents have been described in the literature and their protocols completely determined. Although this technique is simple to use on a laboratory scale, precipitation techniques encounter real problems when one wishes to move up to an industrial scale process.
The main problem, on top of the initial cost of the raw material, is the production of important quantities of polluting liquid effluents. For example, ammonium sulfate, a very commonly used precipitating agent, generates liquid effluent that contains very high amounts of nitrogen, causing retreatment problems on an industrial scale where nitrogen emissions in liquid effluents are severely restricted. Furthermore, where purification of complex molecules is involved, for example with proteins, precipitation can cause resolubilisation problems of the proteinaceous pellet. This tends to make resolubilisation very lengthy and requires fine tuning the buffer conditions, with the risk that the protein undergoes a conformational change leading to loss of the desired properties of the molecule, such as binding capacity, enzymatic activity and the like. In such a case, a refolding step is often necessary, and since refolding is nearly never complete, this results in low yield.

### Expanded bed chromatography

This technique appears to be an alternative for clarification, concentration and partial purification of a target molecule, all in one step. An impurity-rich extract with a high solid particle count can be directly injected onto a column containing a matrix of large diameter beads in an expanded mode. The expanded mode is obtained through stabilized suspension of the beads in the column, by applying an appropriate flow rate. This technique is currently used to produce industrial scale recombinant human serum albumin protein.
As the technique is relatively recent, little data is available for evaluating the global implications of using it, especially with respect to running costs, reproducibility and repeatability, and system maintenance.
Apart from the installation cost of the system, numerous parameters have to be adjusted very precisely, in order to optimize the capture of the target molecule, among which :
- the flow rate required for expansion, which is a function of the nature and turbidity of the injected extract ;
- the operational life of the matrix, dependent on the quantity and nature of the impurities present in the extract ;
- the very low granulometry (less than 50 µm) of the particles present in the injected extract ;
- the ratio of applied rate flow/adsorption kinetics of the protein on the matrix to be used.
All of these parameters lead to a relatively long development phase before the process can run economically on an industrial scale.

The document US 2 626 888 describes the adsorption of vitamin B12 onto diatomaceous earth, but without any indication of the details.

**The document FR 2 467 214** (Ajinomoto and Japan Chemical Research) describes the use of diatomaceous earth for purifying erythropoietin from urine after adjustment of the pH. Amongst the various supports described in this document, (chitosan, XAD7 adsorbant resin, Hyflo super-cell diatomaceous earth), the latter gives the worst recovery yield (50.8% compared to 90.8% and 79% respectively). No data is given as to the yield of adsorption. The ratio of earth to sample is about 1%. Although this document clearly states that the process relates to purification of erythropoietin from humain urine. the latter is not considered to be a complex media compared to plant extracts or other host cell extracts that are processed. Furthermore, no filtration effect is desired or carried out and no reference is made in this document about the filtration technique used on a large scale. Nowhere in this document is there any mention of extrapolation of the system described up to large scale industrial production. The applicants conclude that the use of the techniques described in this document is clearly as an elution technique, with an alcoholic solvant if adsorption occurs on an adsorbant resin, or an alkaline solution when the adsorbant is chitosan. When earth is used, elution takes place through pH modification.

**The document EP 0 480 525** (centro de Ingeniera y biotechnologia) describes a method of producing Hepatitus B surface antigen (HEP B) that is active in vaccinal preparations. The recombinant molecule is expressed in *Pichia Pastoris.* Adsorption occurs at acid pH (pH 3 to 5), with elution of the contaminants by washing and desorption using an alkaline solution at pH 7.5 to 9. A chaotropic agent is used in the lysis buffer. The diatomaceous earth used is Celite (high flow supercell, Fluka), to yield roughly 0.35 g of antigen per kilo of matrix. Purity of the obtained protein is about 40 to 50%, with no denaturing because mild elution conditions are used. The elution yield is approximately 30%. Only example 3 cites the use of diatomaceous earth. The earth is recovered using centrifugation, filtration, and decanting.

**The document US 5 075 430** (Biorad) describes the purification of DNA from bacterial lysates with immobilisation in the presence of chaotropes, followed by washing with alcoholic solution and elution in water or weakly saline solution. The earth used is Celite (superfine super floss. 560, 545), which is an activated earth. This document also gives an explanation as to the different types of earth available (type, granulometry, importance of activation). No retention of proteins is expected because of the presence of chaotropes in high concentration, whereby the ratio of earth to sample is 9 mg of DNA per gram of earth. A yield of 90% is obtained with a desorption volume of 1/10. Thus this document only discloses the use of earth uniquely for the extraction and purification of DNA, in the presence of chaotropes.

**The document US 4 690 892** (Union Carbide Corp) describes a method for phase recycling in liquid/multiphase liquid extraction processes. Some of the supports mentioned include titanium oxide, aluminates, zeolites, calcinated aluminosilicates. The intended targets described are proteases, amylases, cellulases, dextranases, invertases, isomerases, lipases, oxidases, pectinases. In this document, the support is only used to adsorb the contaminating proteins, and nothing is stated about desorption.

The document US 4 309 505 (CPC International Inc.) describes how to increase the production of fructose transferase by improving culture conditions. The patent is essentially focussed on the optimisation of the culture conditions, including adsorption of the enzyme onto diatomaceous earth, addition of earth to the fermentation media, addition of solvant (acetone or 2-propanol) leading to precipitation of the protein and adsorption onto the earth, no desorption (used as a biocatalyst).

Unfortunately, diatomaceous earths are known to have a certain number of disadvantages, among which :
- weak specificity
- variable chemical composition
- variable granulometry
- limited pH range, typically only in acid and neutral pH
- weak ionic capacity
- low surface area, typically around 10⁻³ kg/m³

The present invention seeks to overcome the known disadvantages of the prior art techniques. Consequently, one object of the invention is a method for prepurifying and concentrating a desired recombinant protein from a complex media, wherein said method comprises the steps of :
- bringing the complex media into contact with at least one diatomaceous earth for a sufficient time to effect adsorption of the desired recombinant protein onto the diatomaceous earth ;
- desorbing the desired prepurified and concentrated recombinant protein from the diatomaceous earth.

Although known for its use as a co-adjuvant for filtration, the diatomaceous earth according to the present invention is also used as a support for adsorption. The applicants have surprisingly discovered that the filtration properties of the earth could be combined with adsorption properties to clarify, concentrate, and prepurify a target protein. Thus the technique can be used to purify proteins, and in particular, recombinant proteins, whatever the expression system used.
In particular, the method of the present invention has the following advantages :
- no precipitating agents are needed, reducing or eliminating the pollutant liquid effluent problem;
- clarification and concentration are possible in a single step ;
- it is cost effective and therefore adapted to use in large scale industrial production ;
- the amount of earth used is very low, i.e. only a few %, as a function of the concentration of the target molecule to be purified ;
- it is possible to carry out intermediate washing steps with organic solvants or aqueous buffers.

In accordance with a preferred embodiment of the invention, the desorption step is carried out in the absence of precipitating agents or chaotropic agents.
The term chaotrope (disorder-maker) originally denoted solutes that stabilized, or destabilized respectively, proteins and membranes. Later it referred to the apparently correlating property of increasing, or decreasing respectively, the structuring of water. Although useful, the terminology may sometimes be misleading as such properties may vary dependent on the circumstances. For example a solute may not always act in the same way at different concentrations or in the presence of macromolecules or gels. Also some solutes with less well-defined properties (e.g. urea) are sometimes classified as chaotropes. Both the extent and strength of hydrogen-bonding may be changed independently by the solute but either of these may be, and has been, used as measures of order-making. It is, however, the effects on the extent of quality hydrogen bonding that is of overriding importance as true chaotropes shift the ES<>CS equilibrium to the right.
Ionic chaotropes should be treated differently from non-ionic solutes. Generally, ionic behavior parallels the Hofmeister series. Large singly charged ions, with low charge density (e.g. H₂PO₄⁻, HSO₄⁻, HCO₃⁻, I⁻, Cl⁻, NO₃⁻, NH₄⁺, Cs⁺, K⁺ and tetramethylammonium ions exhibiting weaker interactions with water than water with itself), are chaotropes. Weakly hydrated ions (chaotropes, K⁺, Rb⁺, Cs⁺, Br⁻, I⁻) may be 'pushed' onto weakly hydrated surfaces by strong water-water interactions with the transition from strong ionic hydration to weak ionic hydration occurring where the strength of the ion-water hydration approximately equals the strength of water-water interactions in bulk solution. The ionic chaotropes, by avoiding interference with water's hydrogen-bonded network, tend to clathrate formation within the less dense (ES) environment. Thus there is agreement with the defining characteristic of a chaotrope in that it partitions selectively into low-density water. Chaotropes break down the hydrogen-bonded network of water, so allowing macromolecules more structural freedom and encouraging protein extension and denaturation. Chaotropes decrease the order of water, increase its surface tension and destabilize macromolecular structures (such as guanidinium chloride and urea at high concentrations). Recent work has shown that urea weakens both hydrogen bonding and hydrophobic interactions. The idiosyncratic behavior of urea may well be due to its concentration-dependent oligomerization; cyclic hydrogen-bonding dimers and oligomers behaving differently from monomers.
Exemplary precipitating agents are, for example, ammonium sulfate, metallic cations such as zinc, barium, cadmium, or compounds containing bulky anions, such as picrate, tungstate, tannate, molybdate, trichloroacetate, perchlorate, sulfosalicylate which are commonly used at laboratory scale to separate proteins, but has the disadvantage that at industrial scale production, large quantities of nitrogen or other polluting compounds or compositions containing pollutant liquid effluents are generated. Other precipitating agents known are organic solvents (such as ethanol, butanol, acetone, methanol), organic compounds (e.g. polyethylene glycol). These precipitating agents, however, often have the disadvantage of denaturing the protein, thus causing yield loss and often loss of protein conformity and activity.
In another preferred embodiment of the present invention, the desorption step is carried out in the absence of pollutant liquid effluents. Such effluents, as have already been mentioned, are those which are for example, subject to regulatory emission control, or the degradation products thereof are subject to regulatory emission control. Exemplary pollutant liquid effluents are those that contain, or degrade to give, for example, nitrogen, phosphorous, halogen or halogen containing compounds, heavy metals, such as lead, tin, cadmium, mercury and their salts.

The invention uses diatomaceous earths. Diatomaceous earth, also known as kieselguhr or diatomite, is a loosely coherent chalk-like sedimentary rock made up mainly of fragments and shells of hydrous silica secreted by diatoms, microscopic one-celled algae. The particles are very fine, and have high surface area. Silica content may be as high as 94%. Due to the intricate structure of the diatom skeletons that form diatomaceous earth, the silica has a very different physical structure from other forms in which it occurs. Diatomaceous earth is available commercially in three forms : natural, calcinated, flux calcinated. Calcinated diatomaceous earth is an earth treated by high temperature calcination at about 980°C. Flux calcinated diatomaceous earth is prepared by the calcination of the natural product in the presence of flux, generally soda ash or sodium chloride. This is also known as activation. This treatment reduces the surface area of the diatom particles, changes the colour from the natural buff to white and makes any impurities insoluble. Flux calcinated diatomaceous earth may be obtained commercially from several sources, e.g. Clarcel, available from CECA ATO, France.
Preferably, the diatomaceous earths are chosen from those consisting of activated, also known as calcinated or flux calcinated earths, and non-activated or natural diatomaceous earths.
Even more preferably, the diatomaceous earths are essentially hydrophobic.
According to one preferred embodiment of the present invention, the diatomaceous earths are non activated or natural and chosen from the group consisting of clarcel CBR and clarcel CBL diatomaceous earths. Preferably, the diatomaceous earth is clarcel CBR₃ or clarcel CBL.
In accordance with yet another preferred embodiment of the present invention, the diatomaceous earth is made up of the following metal oxides : SiO₂, Al₂O₃, Fe₂O₃, TiO₂, CaO, MgO, K₂O, Na₂O.
The present applicants have discovered that most favorable, surprising and advantageous results are obtained with the method of the present invention, when the diatomaceous earth has a granulometry substantially of about 10 to about 20 microns. This means that the earths used in the present invention may have a granulometry varying from about 1 µm to about 500µm, with approximately 97% of the earth having a particle size greater than 1µm, and preferably from about 1µm to 350µm. even more preferably from about 1µm to 200µm. The majority of the particles in the most preferred earths used in accordance with the invention have a size of from about 2µm to about 100µm.

In a preferred embodiment of the present invention, the earth is pre-wetted before being brought into contact with the complex media. The applicants have surprisingly discovered that this enables a reduction in the polarity of the earth, and also increases repeatability of the adsorption and desorption steps of the method of the present invention. Preferably, the earth is pre-wetted with water, and even more preferably with deionized water.
In accordance with the present invention, a complex media can be whole cells, cell parts, in the dry or wet state, for example, cell extracts or lysates, cell cultures, structured or unstructured tissue, organs, and the like. Preferably, the complex media is a solid or substantially solid and is substantially made up of cells or cell parts in the dry state. More preferably, the complex media is selected from the group comprising cells or cell parts from animals, humans, yeasts, bacteria, insects, fungi, and plants. When plant cells are the complex media of choice, said complex media is selected from the group consisting of plant leaves, plant stems, flowers, plant pollen, plant seeds, plant cotyledons, plant roots, and plant reproductive organs.
Where plants are chosen as the complex media, angiosperm plants are the preferred plant type. Such plants can be monocotyledonous or dicotyledonous plants. Exemplary dicotyledonous plants are tobacco, lettuce, tomato, and plants of the Solanaceae family, sunflower, safflower, rape, plants of the Crucifera family, cucmber, melon, courgette, zucchini, pumpkin, plants of the Cucurbitaceae family, cabbage, radish, cauliflower, plants of the Brassica family, carrots, lucerne, canola, and arabidopsis, and the like. Exemplary monocotyledonous plants are those members of the Graminae family, such as barley, wheat, maize, oats, sorgho, rice, and the like.
In a preferred embodiment, the complex media is tobacco leaf.
In another preferred embodiment, the complex media is corn (maize) seed.
In yet another preferred embodiment, the complex media is corn seed and the seed is not degermed. Indeed, the inventors have discovered that very good yield can be obtained with maize that has not been degermed, since the undegermed maize retains lipids and the like that envelope the target protein and facilitate its adsorption to the diatomaceous earth.
In still yet another preferred embodiment, the complex media is corn seed and the seed is degermed.

In a most preferred embodiment, the complex media is ground. For example, leaves can be ground cryogenically, e.g. in liquid nitrogen, or frozen by dry freezing, and then ground, and
seed can be ground to form a fine flour.
In another preferred embodiment, the complex media has a granulometry comprised between about 200 microns and about 5mm.
In another preferred embodiment of the present invention, the complex media is a solid or substantially solid, and undergoes a steeping step in a steeping buffer before bringing it into contact with the at least one diatomaceous earth. Preferably, the earth is pre-wetted with the steeping buffer or water before the complex media undergoes the steeping step. The steeping buffer can be a liquid salt-based buffer with a given pH, ionic strength and preferably with a given detergent, designed to bring a solid phase into contact with a liquid phase in order to draw out into the liquid phase the molecules that are of interest from within the solid phase.
Advantageously, the solid phase that is formed is separated off from the liquid phase, e.g. by filtration or decanting.
As mentioned previously, the complex media is brought into contact with the at least one diatomaceous earth for effecting adsorption. The time allotted for this adsorption has been advantageously found to be comprised between 5 minutes to 60 minutes.
In a preferred embodiment of the present invention, the at least one diatomaceous earth is used in an amount comprising 0.5% w/v to 2% w/v.
In accordance with an advantageously preferred embodiment of the invention, the desorption is carried out for a period of time comprised between 5 minutes to 60 minutes.
In a most preferred embodiment, the desorption is carried out at a pH comprised between 2 and 10, preferably at between pH 2.5 to pH 8.
The applicants have determined that through the use of the diatomaceous earths of the present invention, it is equally possible to reduce the volume of desorption buffer used for concentrating the target protein, which has the beneficial effect of reducing overall liquid volumes for the globality of the method. Accordingly, the desorption is preferably carried out with about 15 to about 20 volumes of a desorption buffer for batch processing, and preferably followed by use of about 5 to about 10 volumes of a desorption buffer for static processing.
Optionally, the at least one diatomaceous earth is washed after the adsorption step and before the desorption step. Such a step enables prepurifying and preconcentration of the target protein and assists in eliminating the interstitial liquid fraction that is adsorbed, as well as contaminants. Furthermore, such a washing step enables elimination of spurious lipids, minerals, undesired protein and various impurities.
In still yet another preferred embodiment, the complex media is treated with at least one flocculating and/or coagulating agent before adsorption. This is advantageous, for example, when the complex media is tobacco leaves, and the target protein is recombinant collagen, since the use of a flocculating and/or coagulating agent in this case enabled an increased filtration flow rate, and improved extraction of the recombinant collagen. Flocculating agents are hydrophilic polymers having a molecular weight varying from 1 million to 30 million, i.e. a degree of polymerization of between 14,000 and 420,000 monomer units. Their water solubility comes from sufficiently strong solvation of the polar groups, either ionic or nonionic, that they contain, so that the various segments of a chain are dissociated. Most flocculating agents are currently based on acrylamide, have by homopolymerization, a nonionic nature and may have, by copolymerization, a cationic or anionic nature, with a degree of ionicity varying between 0% and 100%. Coagulating agents are currently characterized by a very high cationic charge to neutralize the negative charges of colloids and a relatively low molecular weight varying from 20000 to 1 million to allow rapid diffusion in the medium and around the particles. Preferably, three large families of coagulants are used, namely quaternary polyamines being organic coagulants, poly diallyldimethyl ammonium chloride (polyDADMAC) obtained by the reaction of allyl chloride with dimethylamine, and dicyandiamide resins obtained by condensation of diacyandiamine with formaldehyde followed by quaternization with ammonium chloride. As will be shown in the detailed description of the invention, the method can be used to extract and prepurify any recombinant protein of interest. Accordingly, the desired recombinant protein is preferably chosen from recombinant proteins having therapeutic and/or nutraceutic activity. By therapeutic activity, it is to be understood that the recombinant protein has a biological activity implicated in, or important to, the treatment of a human or animal pathological condition. By nutraceutic activity, it is to be understood that the recombinant protein has a biological activity implicated in. or important to the improvement or supplement of human or animal nutrition. For example, such therapeutic proteins can be hemin proteins, antibodies, antibody fragments, collagen polypeptides, iron binding proteins, immunostimulatory or immunomodulatory proteins, digestive enzymes, immune response stimulating glycoproteins, vascular proteins, cerebral proteins and neuroproteins.
Exemplary recombinant proteins that can be purified according to the method of the present invention can be chosen from the group consisting of gastric lipases, pancreatic lipases, lactoferrins, collagens, monoclonal antibodies, hemoglobins, fibrinogens, monokunines, bikunines.

### Brief description of the figures

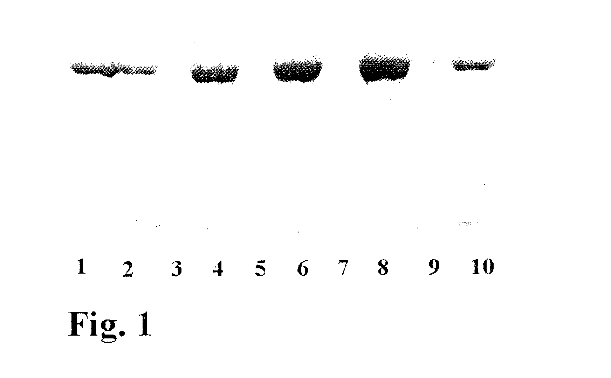

Figure 1 represents a blue Coomassie Gel of the extraction and purification according to the invention of recombinant collagen produced in tobacco plants. In this figure the lanes are identified as follows :
- Lane 1 : recombinant collagen control
- Lane 2 : filtered macerate
- Lane 3 : filtrate collected after adsorption of filtered macerate with 1.5% w/v of diatomaceous earth and vigorous agitation for 30 minutes.
- Lane 4 : desorbate collected after desorption with desorption buffer (a)
- Lane 5 : filtrate collected as lane 3
- Lane 6 : desorbate collected after desorption with desorption buffer (b)
- Lane 7 : filtrate collected as in lane 3
- Lane 8 : desorbate collected after desorption with desorption buffer (c)
- Lane 9 : filtrate collected as in lane 3
- Lane 10 : desorbate collected after desorption with desorption buffer (d)

### Detailed description of the invention

The applicants have studied several parameters for the development of this technique, in order to ascertain a range of operating conditions, including what the applicants consider to be the optimal conditions:
- for the crude extract
   - pH
   - type and concentration of the salts present
   - presence of impurities or contaminants
   - turbidity
   - temperature
   - homogeneity of the extract, i.e. quality of agitation/stirring
- for the diatomaceous earths
   - pre-wetting
   - number of available sites
   - surface charge (activated/non activated with sodium carbonate)
   - contact time (adsorption/desorption)
   - adsorption surface specificity
   - concentration
   - composition (presence of various metal oxides)
   - particle size and permeability
- for the targe molecule to be purified (e.g. proteins)
   - primary sequence
   - net charge
   - hydrophobicity
   - molecular weight
   - isoelectric point
   - enzymatic activity (where enzymes are the target)
   - known specific affinity sites

The number of parameters that has to be considered can account for the difficulty of developing this technique as a clarification and concentration step, enabling the preparation of an extract exempt of solid particles, and partially purified and concentrated.

### 3-2) Methodology

### Pre-wetting

It has been found by the present applicants that this step, although simple to carry out, can influence both the adsorption and desorption of the protein onto and from the support. On the one hand, pre-wetting enables a reduction in the polarity of the support. This step thus permits modulation of the phenomena and where such a phenomena is unwanted, to completely suppress it. On the other hand, the pre-wetting step increases desorption yield which is linked to solvation of the support.
Thus, before use, the diatomaceous anhydrous earth is preferably pre-wetted, either preferably with water, or in the steeping buffer.

### Adsorption

The earth can be brought into contact with the liquid sample containing the protein to be purified, preferably without modifying the conditions used during steeping, i.e. without for example adjusting the pH, adding chaotropes, or precipitating agents. In the case of recombinant proteins, where the amount of target protein present in the complex media is relatively low, the quantity of earth used can also be low, preferably lower than 5% by weight, more preferably between 0.5% and 2% by weight. Optimal contact time can be determined during screening and is preferably at most 2 hours, and preferably between 5 and 60 min. For example, in the case of a recombinant dog gastric lipase, the contact time is voluntarily relatively short, since otherwise the protein tends to optimise surface contact with the support, thereby reducing the efficiency of subsequent desorption. The mixture is maintained with stirring or equivalent agitation means to allow for maximum contact between the protein and the diatomaceous earth. After adsorption, the mixture is separated by appropriate techniques, to retain the earth on which the protein has adsorbed. At lab scale, an example of such a technique would be centrifugation, then filtration and natural decantation. On an industrial scale, one can proceed via centrifugation, filtration on plate filters or rotating filters under vacuum.

### Washing

After separation of the solid fraction having adsorbed the target molecule from the crude extract, the earth can be washed with buffer adapted or chosen to eliminate any remaining interstitial crude extract liquid and/or any potentially remaining impurities such as lipids, proteins, mineral salts and the like. Depending on the type and amount of impurity to be eliminated, the wash buffer can be adapted in volume, say, up to 25 volumes, preferably. from 5 to 15 volumes, and in composition, whereby water can be used to eliminate impurities like DNA and unwanted protein, and organic solvants, preferably diluted, can be used to eliminate pigments. The washing step can be carried out as a batch step or more preferably statically.

### Desorption

After adsorption, and an optional washing step. the earth is brought into contact with a desorption buffer, the latter being a buffer that will enable the best acceptable desorption yields. The earth is resuspended in the buffer and agitated until homogeneously dispersed. The volume of desorption buffer used is preferably the smallest possible quantity, for example, from 10 to 30 volumes of buffer with respect to the quantity of dry earth, and preferably from 10 to 20 volumes, and most preferably from 15 to 20 volumes. The desorption time can be optimized during screening, but will generally be relatively short, preferably at most 2 hours, even more preferably from 5 to 60 minutes. After desorption, the solid/liquid mixture is separated using techniques described as above for the separation of the adsorbed target molecule from the crude extract. The dry cake obtained can again be brought into contact with the desorption buffer, once more or several times, either in batches or preferably statically, in a small volume, preferably from 1 to 5 volumes of desorption buffer. This second desorption step which is statically processed, is preferred because it increases the desorption yield in a significant way, without causing a too great a dilution of the desorbed sample.

### 4)Examples

### 4-1) Recombinant lipase extracted from corn (example 1)

Corn grain expressing the gene coding for dog gastric lipase was finely ground and left to macerate for 16 hours, under agitation and at ambient temperature, in 16 volumes of steeping buffer consisting of glycine 50 mM, NaCl 250 mM and Triton X100 1 mM, the pH being adjusted to 2.5 by the addition of hydrochloric acid or sulfamic acid, the latter being preferred since this is less corrosive on the extraction equipment.

After steeping, a rough filtration was carried out on Miracloth in order to eliminate the largest particles. The pH was then adjusted to 4.0 by the addition of sodium acetate salts. Non activated diatomaceous earth, i.e. earth that had not been activated by sodium carbonate treatment, (Clarcel type CBL, available from CECA ATO), was pre-wetted in 10 volumes of tap water and then dried on a Büchner filter, and added to the macerated extract at pH 4.0, at a concentration of 2% w/v, dry weight. The mixture was agitated for 15 minutes, allowing for adsorption of the recombinant lipase onto the diatomaceous earth. After filtration, for example, on a Büchner filter at lab scale and on plate filters, or rotating filters under vacuum, or tubular filters at an industrial scale, the earth cake obtained is resuspended in 20 volumes desorption buffer. The buffer had the following composition :
- glycine 50 mM;
- NaCl 5 mM;
- Triton X100 2 mM;
- pH adjusted to 2.5 by addition of hydrochloric acid

The mixture was agitated for 30 minutes, then filtered on one of the systems described previously, with the cake being rinsed twice with 5 volumes each of desorption buffer. The three aliquots obtained were pooled and could be used directly in a complementary purification step, for example, on a chromatography column or by ultrafiltration.

In the aforementioned operating conditions, it was possible to obtain a perfectly clear sample that was very faintly colored. The adsorption and desorption yields were close to 100%, with a concentration factor of 1.67.

### 4-2) Recombinant lipase extracted from corn (example 2)

The same operating method as for example 1 was used. up to mixing the crude extract with the diatomaceous earth. After filtration, the earth cake was statically washed with 10 volumes of demineralized water and then resuspended in 15 volumes of desorption buffer having the following composition :
- glycine 50 mM;
- NaCl 5 mM;
- Triton X100 2 mM;
- pH adjusted to 2,5 by addition of hydrochloric acid

The mixture was maintained under agitation for 30 minutes, then filtered as described previously. The cake obtained was rinsed with 5 volumes of desorption buffer. The two aliquots obtained were pooled and could be used directly in a complementary purification step.
In the conditions described above, the sample obtained is perfectly clear and only very faintly colored. The adsorption and desorption yields were close to 100%. No recombinant lipase was detected in the unretained fraction or the rinsing fraction, indicating no loss of recombinant lipase. The concentration factor was 2.5.

### 4-3) Recombinant lipase extracted from corn (example 3)

Example 3 is identical to example 2, except for the composition of the desorption buffer, which was as follows :
- glycine 50 mM;
- NaCl 75 mM;
- Triton X100 2 mM;
- pH adjusted to 2.5 by addition of hydrochloric acid.
Under these conditions, the sample obtained was perfectly clear and only very faintly colored. No lipase was detected in the unretained fraction or the rinsing fraction. The adsorption and desorption yields were close to 100%, and the concentration factor 2.5. The increase in NaCl concentration increases conductivity of the sample, which can then be used advantageously in a subsequent ion exchange chromatography purification step.

### 4-4) Recombinant lipase extracted from corn (example 4)

Example 3 is identical to example 1, with the exception of the desorption composition, which was as follows :
- deionized water, or deionized water adjusted to pH 3.5.

Under these conditions, the adsorption yield was close to 100%, but the desorption yield was very low, 3.55% and 2.2% respectively.

### 4-5) Recombinant lipase extracted from corn (example 5)

Using the same conditions as in example 1, but with corn grain that had had the germ removed, the sample obtained is perfectly clear and only very faintly colored. In this case, the adsorption and desorption yields were close to 75% and 65% respectively.

### 4-6) Recombinant lipase extracted from corn (example 6)

Using the same conditions as in example 1, with the exception that the earth was not subjected to a prehumidification step, the sample obtained was perfectly clear and very faintly colored. The adsorption yield remained close to 100%, but the desorption yield was at most 85%.

### 4-7) Recombinant lipase extracted from corn (example 7)

To determine the influence of the environmental media, reconstructions were carried out by bringing purified recombinant lipase powder in contact within water, buffer (described in example 1, pH3.5) or crude extract of non transformed maize (adjusted pH).

| | Lipase adsorption (%) | Lipase desorption |
|---|---|---|
| Water | 100 | Very little |
| Buffer (described in example 1) | 85 | trace |
| Crude extract of non transformed maize | 52 | Total desorption |

These results indicate that lipase desorption is dependent on the environment of the complex media.

### 4-8) Recombinant collagen extracted from tobacco leaves (example 8)

Tobacco leaves expressing the gene coding for collagen type I were cryogenically ground and left to macerate for half an hour, under agitation at a temperature of 4°C, in 5 volumes of steeping buffer having the following composition :
- acetic acid 500 mM;
- NaCl 200 mM;
- EDTA 2.5 mM;
- the pH being between about 2.5 and about 2.8

After steeping, a rough filtration on 10µ cloth was carried out to eliminate the largest solid particles. Diatomaceous earth of the type Clarcel CBL was added to the crude extract, at a concentration of 2% w/v, dry weight. The mixture was agitated for 15 minutes to allow for adsorption of the recombinant protein onto the earth. After filtration, the earth cake was resuspended in 20 volumes of desorption buffer having the following composition :
- glycine 50 mM pH 2.5;
- NaCl 5 mM;
- Triton X 100 2 mM

The mixture was agitated for 30 minutes and then filtered on one of the systems mentioned previously, after which the cake was rinsed with 5 volumes of desorption buffer. The two aliquots collected were pooled and could be used directly in a complementary purification step.

Under these conditions, the sample obtained was perfectly clear, and only faintly colored. The adsorption and desorption yields were close to 100%, and the concentration factor was 2.5.

### 4-9) Recombinant collagen extracted from tobacco leaves (example 9)

The same conditions were used as for example 7, except that the desorption buffer had the following composition :
- glycine 50 mM;
- NaCl 75 mM;
- Triton X 100 à 2 mM;
- pH 2.5

Under these conditions, the adsorption yield remained close to 100%, but the desorption yields dropped to about 50%.

### 4-10) Recombinant Human Serum Albumin (HSA) extracted from tobacco cell culture media

### Quantitative test

Humain Serum Albumin (HSA) was solubilized at a concentration of 0.1 mg/mL in a 200 mM acetate buffer, at pH 4.0, containing 250 mM NaCl, 1 mM EDTA and 1 mM Triton X100.

Diatomaceous earth at 2% w/v (Clarcel CBR₃ or CBL) was added to the solution, the mixture then being placed under agitation for 30 min.
After filtration, the earth cake was resuspended in 10 volumes of desorption buffer having the following composition :
- phosphate buffer 200 mM;
- NaCl 250 mM;
- EDTA 1 mM;
- Triton X 100 à 1 mM;
- pH 8.0

The mixture was agitated for 15 minutes and then filtered.

Under these conditions, for the two types of earth used (CBR₃ and CBL), the adsorption yield was respectively 85% for CBR₃ and 98% CBL, with a desorption yield that was respectively 83% for CBR₃ and 75% for CBL, without static rinsing. The concentration factor was 5.

### Qualitative test

Tobacco cell culture media, containing secreted recombinant HSA was used as the starting complex media. Such media are generally based on Murashige-Skoog growth media and was harvested after a several days of cell growth. The media was yellowish in color, rich in polysaccharides, mineral salts, pigments and endogenous secreted proteins. Upon harvesting, the media had a pH of 5.21. Addition of 1 M hydrochloric acid enabled adjustment of the pH to 4.0. Diatomaceous earth of the type Clarcel CBR₃ and CBL were added to separate samples of culture media, at a concentration of 2% w/v. The mixtures were agitated for 30 minutes at ambient temperature. Where the mixtures are complex, a washing step can be carried out with acetate buffer as described previously, to eliminate non adsorbed impurities.
The mixture was filtered and the earth cake resuspended as described previously.
Gel electrophoresis analyses using SDS-PAGE and Western blots showed that the recombinant HSA was completely retained on the diatomaceous earths used and desorbed at a good yield, although the exact determination of the yield was difficult in view of the very low quantities of target molecule in the culture media.

### 5)Mechanism

The nature of interactions involved is complex and strongly depends on the properties and characteristics of the diatomaceous earth used and the target molecule to be purified.

### 5-1) Diatomaceous earth

Diatomaceous earth is available as a powder or as granulates, i.e. rigid particles, rough surfaced, capable of forming a stable heap and resisting compression. They are highly chemically inert, with low density and a very high cavity ratio.
Their chemical composition varies, but generally, they are composed mainly of a silicon dioxide (SiO₂) crystalline skeleton, in which other metal oxides of various different types are present, e.g. Al₂O₃, CaO, Na₂O Fe₂O₃, K₂O, MgO, MnO, P₂O₅ and a few percent of variables. The silicon dioxide in the earth tends to make them hydrophilic.
Diatomaceous earths thus have a large adsorbant capacity with respect to polar compounds, involving electrostatic dipole/dipole type interactions. but also with respect to hydrophobic molecules present in aqueous medium. Furthermore, the metals present as oxides, but inert, act as Lewis acids and may be involved in the retention phenomena of the target molecule on the support.
There are basically two types of earth, a form that has been activated with sodium carbonate, of a generally white aspect, and a non-activated form, of a generally pinkish or pinkish brown aspect. This difference, that occurs during the production of the earths, enables modulation of the electrostatic interactions, depending on the earth used, but also hydrophobic interactions.

### 5-2) Proteins (in accordance with example 4)

- Lipase

### Laboratory scale

After prehumidification of the earth, thereby reducing its polarity, the earth is brought into contact with the protein, which is present in an alkalinised aqueous buffer at pH 4.0 at a concentration of 250 mM NaCl. The recombinant protein, previously expressed in transgenic corn (maize) seed, was present in a flour obtained by grinding the maize seed, which was then steeped in the aqueous buffer to obtain a suspension. At this pH, the net charge of the protein is close to zero (Pi close to 5). The lipase is an esterase, having an active site capable of hydrolysing fatty acids. The region where this active site is situated can be considered as relatively hydrophobic. Compared to the aqueous buffer, the earth represents an environment that is more favorable to the protein. The protein is thus adsorbed onto the surface of the earth at the hydrophobic region. It has been demonstrated that lipase binding to a silica support such as Celite, for use as a biocatalyst, in the presence of an apolar solvent enables recovery of greater enzymatic activity than in aqueous solution alone. This tends to indicate that the apolar solvent orients the active hydrophobic site towards the exterior whereas in aqueous buffer alone, the active site appears to be oriented inwardly towards the surface of the support. Washing with water eliminates impurities without eluting the protein, as observed in experiments. The elution is carried out by increasing the concentration of Triton X100 in the buffer, whilst at the same time lowering the concentration in salt NaCl (from 250 mM to 5 mM). It has been observed that the presence of sodium chloride is necessary in order to obtain good desorption yield, whereby TX100 alone gave a desorption yield of about 40%, NaCl alone gave a desorption yield of about 45%, and both together gave an absorption yield in excess of 95%.
Whereas the adsorption seems to be controlled uniquely by the hydrophobic interactions, desorption seems to be influenced by both hydrophobic interactions (importance of the role of Triton X100), but also by electrostatic interactions (importance of NaCl).

### Conclusions LIPASE :

Optimal Desorption pH : 2.5
Optimal Desorption Time : 30 minutes
These conditions give optimal desorption kinetics.
Optimal desorption volume : 20V buffer (batch) followed by 5V buffer (static)
Optimal grain usage : whole grain, not degermed (maize, monocots).
Prehumidification of the earth (maize derived macerate), prehumidification by direct addition to crude extract.
Optimal conditions (maize lipase):
Adsorption on 2%) CBL, with 5V H₂O w/v earth, for 15 mins
Filtration without pre-layer
Static rinsing 10V H₂O w/v earth
Desorption in 20 V buffer (batch) for 30 minutes, followed by 5V buffer (static)
Buffer : glycine 50 mM, NaCl 75 mM, Triton X100 2 mM, pH 2.5

### Pilot scale

Extraction at the pilot scale level was performed on 70 kg undegermed grains of transformed maize. The procedure was similar to that used at the laboratory scale level.

| ***Sample*** | ***Vol(l)*** | ***Activity (TC4 U*/*ml)*** | ***Total activity (×1000)*** | ***Yield (Lipase activity %)*** | ***Mg*/*ml Protein*** | ***Total protein (g)*** | ***Specific activity (TC4 U*/*mg)*** |
|---|---|---|---|---|---|---|---|
| EB | 961.1 | 22.2 | 21336.42 | 100.0 | 2.37 | 2277.807 | 9.4 |
| EBc | 961.1 | 21.8 | 20951.98 | 98.2 | 1.52 | 1460.872 | 14.3 |
| EB pH4 | 912 | 22.7 | 20702.4 | 101.2 | | | |
| F1 | 908.6 | 0.4 | 363.44 | 1.8 | 1.19 | 1081.234 | 40.3 |
| F2 pool | 450.2 | 37.2 | 16747.44 | 80.9 | 0.29 | 130.558 | 128.3 |
| EB: crude extract, EBc: centrifuged crude extract, EB pH4: alkalinised crude extract, F1: filtrate collected after adsorption, F2 pool: desorbate collected after desorption | | | | | | | |

The obtained results show :
- During the adsorption step, the lipase is retained whereas 47.5% of contaminant proteins remain in F1.
- After desorption step, 5.7% of total proteins (EB) were found in the desorbate. This means that this step allows the elimination of about 95% of contaminant proteins and the increase of specific activity (factor 22).

Comparable results have been obtained at the industrial scale level with batches of about 700 kg transformed maize whole grains.
- HSA

For HSA, the protein present in a aqueous buffer at pH 4.0, with a net charge close to zero is adsorbed onto earth that has not been previously wetted before contact, thereby having a high polarity. By changing the pH from 4 to 7, the net charge of the protein switches to become globally negative, leading to repulsion with the silanol groups, and therefore elution of the protein. In this case, the adsorption and desorption seem to be influenced by electrostatic interactions of dipole/dipole type.
- Collagen

### Laboratory scale level

The recombinant collagen present in the aqueous salt buffer at pH 2.5, with a net positive charge is brought into contact with CBL diatomaceous earth that has not been pre-wetted. The adsorption was carried out on Miracloth prefiltered macerate and brought into contact with 1.5% w/v, under vigorous agitation for 30 minutes. After adsorption, Buchner filtration was carried out to separate the earth and adsorbed protein from the remaining liquid. This liquid was retained and analysed for protein content.
Desorption was obtained by adding 20V buffer of varying composition, under vigorous agitation for 30 minutes :
a) glycine 50 mM, NaCl 400 mM , Triton X100 1 mM, at pH 2.5;
b) glycine 50 mM, NaCl 400 mM, Triton X100 2 mM, at pH 2.5;
c) glycine 50 mM, NaCl 5 mM, Triton X 100 2 mM, at pH 2.5;
d)phosphate buffer 50 mM, NaCl 5 mM, Triton X100 2 mM, at pH 8;

After desorption, a Buchner filtration was carried out to separate the desorbed proteins in aqueous solution from the earth. This liquid was then analysed for protein content. It was observed that the above process steps enabled both a concentration of the sample and its prepurification. of industrial scale interest.
In this case, the adsorption seems due mainly to electrostatic interactions of the dipole/dipole type whereas desorption appears to be due to mainly to hydrophobic interactions.

### Pilot scale level

Extraction at the pilot scale level was performed on 75 kg transformed tobacco leaves. The procedure was similar to that used at the laboratory scale level.
Similar results have been obtained at the pilot scale level comparatively to the laboratory scale level.

## Claims

1. Method for prepurifying and concentrating a desired recombinant protein from a complex media, wherein said method comprises the steps of:
- bringing the complex media into contact with at least one diatomaceous earth for a sufficient time to effect adsorption of the desired recombinant protein onto the diatomaceous earth :
- desorbing the desired prepurified and concentrated recombinant protein from the diatomaceous earth.

2. Method according to claim 1, wherein the desorption step is carried out in the absence of precipitating agents or chaotropic agents.

3. Method according to claim 1, wherein the desorption step is carried out in the absence of pollutant liquid effluents.

4. Method according to claim 1, wherein the diatomaceous earths are chosen from those consisting of activated and non-activated diatomaceous earths.

5. Method according to claim 1, wherein the complex media is a solid or substantially solid, and undergoes a steeping step in a steeping buffer before bringing it into contact with the at least one diatomaceous earth.

6. Method according to claim 1, wherein the complex media is a solid or substantially solid and is substantially made up of cells or cell parts in the dry state.

7. Method according to claim 1, wherein the complex media is selected from the group comprising cell cultures, cell lysates, structured or unstructured tissue, organs, and the like.

8. Method according to claim 1, wherein the complex media is selected from the group comprising cells or cell parts from animals, humans, yeasts, bacteria, insects, fungi, and plants.

9. Method according to claim 1, wherein the complex media is selected from the group consisting of plant leaves, plant stems, flowers, plant pollen, plant seeds, plant cotyledons, plant roots, and plant reproductive organs.

10. Method according to claim 1, wherein the complex media is selected from angiosperm plants.

11. Method according to claim 1, wherein the complex media is selected from monocotyledonous or dicotyledonous plants.

12. Method according to claim 1, wherein the complex media is tobacco leaf.

13. Method according to claim 1, wherein the complex media is corn (maize) seed.

14. Method according to claim 1, wherein the complex media is ground.

15. Method according to claim 1, wherein the complex media has a granulometry comprised between about 200 microns and about 5 mm.

16. Method according to claim 1, wherein the diatomaceous earths are essentially hydrophobic.

17. Method according to claim 1, wherein the diatomaceous earths are non activated and chosen from the group consisting of clarcel CBR and clarcel CBL diatomaceous earths.

18. Method according to claim 1, wherein the diatomaceous earth is clarcel CBR₃ or clarcel CBL.

19. Method according to claim 1, wherein the diatomaceous earth is made up of the following metal oxides : SiO₂, Al₂O₃, Fe₂O₃, TiO₂, CaO, MgO, K₂O, Na₂O.

20. Method according to claim 1, wherein the diatomaceous earth has a granulometry of about 10 to about 20 microns.

21. Method according to claim 1, wherein the earth is pre-wetted before being brought into contact with the complex media.

22. Method according to claim 1, wherein the earth is pre-wetted with water or the steeping buffer.

23. Method according to claim 1, wherein the earth is pre-wetted with deionized water.

24. Method according to claim 1, wherein the complex media is corn seed and the seed is not degermed.

25. Method according to claim 1, wherein the complex media is corn seed and the seed is degermed.

26. Method according to claim 1. wherein the complex media is brought into contact with the at least one earth for effecting adsorption for a period of time comprised between 5 minutes to 60 minutes.

27. Method according to claim 1. wherein the at least one diatomaceous earth is used in an amount comprising 0.5% w/v to 2% w/v.

28. Method according to claim 1, wherein the desorption is carried out for a period of time comprised between 5 minutes to 60 minutes.

29. Method according to claim 1, wherein the desorption is carried out at a pH comprised between 2 and 10, preferably at between pH 2.5 to pH8.

30. Method according to claim 1, wherein the desorption is carried out with about 15 to about 20 volumes of a desorption buffer for batch processing and preferably followed by use of about 5 to 10 volumes of desorption buffer for static processing.

31. Method according to claim 1, wherein the at least one diatomaceous earth is washed after the adsorption step and before the desorption step.

32. Method according to claim 1, wherein the complex media is treated with at least one floculating agent before adsorption.
